# EUROPEAN PATENT APPLICATION

(11) **EP 1 704 862 A1**
(43) Date of publication of application: **27.09.2006**
(21) Application number: 05102414.9
(22) Date of filing: 24.03.2005
(51) Int. Cl.: A61K 31/4965, A61P 19/02, A61P 29/00, A61P 1/04, A61P 1/00, A61P 25/28

(54) **2,5-bis(tetrahydroxybutyl)pyrazines for the treatment of osteoarthritis and rheumatoid arthritis**

(71) Applicant: ROTTAPHARM S.P.A., 20122 Milano (IT)
(72) Inventor: Giordani, Antonio, 27100, Pavia (IT); Letari, Ornella, 20131, Milano (IT); Persiani, Stefano, 20020, Arese (Milano) (IT); Artusi, Roberto, 20017, RHO (Milano) (IT); Peris, Walter, 20159, Milano (IT); Caselli, Gianfranco, 20129, Milano (IT); Rovati, Lucio Claudio, 20052, Monza (Milano) (IT)
(74) Representative: Rambelli, Paolo

(57) **Abstract**

Use in the prevention and/or in the treatment of Osteoarthritis and Rheumatoid Arthritis of 2,5-Bis(tetrahydroxybutyl)pyrazines, compounds of formula (I), is disclosed. Compounds of the invention act simultaneously on cartilage matrix degradation and on nitric oxide and prostaglandin E2 production.

## Description

### Field of the invention

This invention relates to the field of therapeutics and in particular relates to methods of treating arthritis, in particular osteoarthritis and rheumatoid arthritis.

### Background of the invention

Osteoarthritis (OA) is a complex degenerative disease that affects a large proportion of the elderly population, characterized by progressive joint degeneration, cartilage degradation and loss of articular function. OA affects more than 50 million people only in the United States and its incidence is expected to increase with the increase of the population average age.

The most of the currently used pharmacological treatment for this disease is largely confined to analgesics, steroidal and especially non-steroidal anti-inflammatory drugs (NSAIDs). These agents modify only the symptoms of the disease rather than the underlying process; in addition, though these drugs have provided an important mean of controlling the inflammation and pain in OA, their application has been overshadowed by the gastrointestinal tract side effects, when considering classical NSAIDs (BV Sweet et al., Am. J. Health Syst. Pharm., 61, 18, 1917-21, 2004) and other side effects when considering the newer COX-2 inhibitors (Mamdani M. et al., Lancet, 363, 1751-6, 2004).

Therefore, the development of new therapies that will be able to modify the pathophysiology of OA is essential.

OA is characterized by pathological changes that occur in the articular cartilage, synovium and subchondral bone leading to pain and loss of joint function.

Although the aetiology of OA is poorly understood, the degradation of cartilage that occurs in this disease is the result of enzymatic cleavage of its structural components.

Cartilage is constituted of chondrocytes and an extracellular matrix that consists of proteoglycans (mainly aggrecan), collagen and water. The interaction between proteoglycans and collagen provides unique structural and physiological properties for cartilage to function in weight bearing and joint motion. Cartilage proteoglycans consist of a protein core with glycosaminoglycan (GAG) side chains; GAGs in human include keratan sulphate, dermatan sulphate, heparan sulphate, and chondroitin sulphate.

GAG components absorb water and provide to the cartilage its characteristic resistance to mechanical stress and constitute a protective layer essential to the joint function.

Healthy cartilage maintains a dynamic equilibrium between processes that produce and processes that degrade the matrix components; in OA, this equilibrium is altered leading to the prevalence of the degenerative process which leads to pronounced matrix degradation, and hence that leads to roughening and fissuring of the cartilage and can eventually result in erosion and/or reaction of the subchondral bone, and mild synovial inflammation.

Aggrecan degradation is one of the main processes of cartilage degeneration, it is due to the catabolic proteolytic cleavage mediated by enzymes known as "aggrecanases" that cleave the GAG side chains. This GAG loss has also been detected in an in vitro model of cartilage injury. Injurious compression of articular cartilage which leads to increased risk for OA is characterized by an initial high rate release of GAG and then followed by collagen degradation.

Accordingly, drugs able to inhibit pathological cartilage degradation by preventing aggrecan degradation, can effectively prevent the cartilage damage progression and thus can be useful pharmacological agents for OA treatment.

Involvement of cytokines in the normal joint and articular cartilage homeostasis, by maintaining the balance between cytokine-mediated anabolic and catabolic processes has been largely documented, as well as the IL-1β induced catabolic cascade which involves many pro-inflammatory enzymes and mediators that are key factors in joint destruction characterizing OA. Thus, even though mechanical stress or injury may provide the initial stimulus for the cascade leading to cartilage degradation, it is the disruption of the balance between cytokine-controlled degradative and reparative processes that ultimately leads to loss of cartilage integrity in OA (J. Pelletier, Osteoarthritis and Cartilage, 12, S63-S-68, 2004). Both the well known enzymes cycloxygenase-2 (COX-2) and prostaglandin E synthase (PGES) involved in the pro-inflammatory prostaglandin E₂ (PGE₂) synthesis, have been shown to be induced by IL-1β produced by synovial cells and chondrocytes (M.Murakami et al., J.Biol Chem., 275, 32783-92, 2000; Kojima F et al., Arthritis Res. Ther., 6, 4, R355-65, 2004).

PGE₂, has many roles in the degenerative processes involved in OA. PGE₂ produced by COX-2/PGES mediated pathway has been demonstrated to enhance IL-1β triggered proteoglycan degradation in OA cartilage, to decrease proteoglycan synthesis and stimulate chondrocytes apoptosis (SB Abramson, Osteoarthritis and Cartilage, 7, 380, 1999).

Moreover, the synovial fluid of patients with OA and rheumatoid arthritis (RA) contains high concentrations of PGE₂, as well as cartilage and chondrocytes from OA patients release more PGE₂ than normal cartilage. Thus, beside mediating pain and inflammation in OA, PGE₂ plays also a key role in cartilage degeneration associated with this disease (M. Hardy et al., Arthritis and Rheumatism, 46, 7, 1789-1803, 2002).

Though nowadays there are no doubts about the key role played by PGE₂ in mediating IL-1β induced joint degeneration in OA, some evidences suggests it is not the only key player.

The lack of significant clinical results in preventing severe disease progression, obtained in OA with COX-2 inhibitors as well as the failure of exogenous PGE₂ to induce proteoglycan degradation in synovial-cartilage co-cultures, when IL-1β is not present, strongly suggest that another IL-1β induced factor is acting along with PGE₂ during cartilage degeneration.

Induction of NO synthesis was demonstrated both in animals and in human articular chondrocytes (J. Stadler et al., 47, 3915-3920, 1991; RM Palmer et al., Biochem. Bioph. Res Comm., 193, 398-405, 1993), and it is well known that inducible nitric oxide synthase (iNOS) is among the pro-inflammatory enzymes involved in several diseases. iNOS can be induced by cytokines and LPS in several cell types; in particular, human chondrocytes are among the few human cell types where NO production is induced in response to LPS alone or to a single cytokine such as IL-1β or TNFα, while other human cell types require a combination of various cytokines for iNOS induction (AR Amin et al., J. Exp. Med., 182:2097-2102, 1995). OA cartilage produces large amounts of NO after iNOS induction by IL-1β, where both its product, NO, and NO by-products represented by reactive oxygen species (ROS), are capable of inducing tissue damage and degeneration. It has been proved that in cartilage NO activates metalloproteinases, inhibits collagen and proteoglycan synthesis and induces chondrocyte apoptosis. NO is involved in the up-regulation of pro-inflammatory cytokine synthesis in the osteoarthritic joints, by reducing the synthesis of the natural IL-1β receptor antagonist (IL-1β RA) and by increasing TNFα production in synovial cells (E. Molianen et al., Osteoarthritis and Cartilage, 9, 597-605, 2001). Finally, it has been recently demonstrated that NO is also able to increase COX₂ activity, thus amplifying the effects highlighted above for the main pro-inflammatory and pro-degradative product, PGE₂, generated by this enzyme. Additional studies have also demonstrated that the induction of COX-2 expression and the consequent increase in PGE₂ is essential for NO-induced chondrocytes apoptosis, since COX-2 inhibition completely blocks the apoptotic phenomenon (JP Pelletier et al, J. Rheumatol., 26, 2002-14, 1999; K. Notoya et al., J. Immunology, 165, 3402-10, 2000). Considering what reported above, it appears that pharmacological agents able to inhibit both PGE₂ and NO production, in chondrocytes stimulated with IL-1β, will be highly effective pharmacological agents for the treatment of OA. These agents, acting at both the pathways that taken together have been shown to be responsible not only for inflammation and pain but also for the tissue degeneration can effectively block the disease progression.

Rheumatoid Arthritis (RA) is a chronic joint inflammatory disease characterized by abnormal synovial proliferation and invasion into adjacent tissues, which also leads to destruction of articular cartilage and bone. This disease affects people of all ages and frequently causes disability and chronic impairments of considerable social cost and impact.

Even though the aetiology of this disease is still unknown, it is generally accepted that RA is a disease involving immunologic processes. Several studies have proven that RA synovial cells release pro-inflammatory prostaglandin (PGE₂) and cytokines, such as IL-1β, TNFα and IL-6, all of which are known to promote the degenerative processes above described for OA (WP. Arend et al., Arthritis & Reum., 33, 305-15, 1990). The mechanisms mediating the cartilage degradation and bone resorption are also in this pathology involving PGE₂ and NO as key players, as pointed out for OA. The role of PGE₂ in this disease has been extensively discussed in the literature (Willoughby DA, Biorheology, 39, 171-9, 2002; CG. Jackson, Expert Opinion Investig. Drugs, 10, 7, 1317-25, 2001; EW Clair, Current Rheumatol. Rep., 149-56, 1999) and it is well documented how inhibition of PGE₂ synthesis can provide valuable therapeutic effect in RA, even though there are serious doubts about effectiveness in preventing tissues degeneration and disease progression when only the prostaglandin pathway is targeted (DA Willoughby et al, Inflammation, 509-19,1998).

Elevated nitrate levels have been found in urine and serum of patients suffering from RA and even higher levels have been detected in the synovial fluid, indicating also in this case the inflamed joint as the NO source. Furthermore, *i*NOS has been detected in synovium and cartilage of RA patients. As previously discussed, also in RA excessive NO production could account for or contribute to the observed cartilage damage by promoting the previously described cartilage degeneration and chondrocyte apoptosis. In addition, it has been proved that NOS inhibitors can reduce the severity of arthritis in animal models (Stefanovic-Racic M et al., Arthritis Rheumatism 7, 1062-1069, 1994; McCartney-Francis N., J. Exp. Med 178, 749-754,1993).

Moreover, it has been recently reported how GAG release can play a relevant role also in RA. Patients with RA have elevated concentrations of GAGs in blood and synovial fluid, and destruction of involved joints in RA patients correlates with high GAGs levels in synovial fluid (J.Y. Wang et al., PNAS, 99, 14362-14367, 2002). Accordingly, compounds acting as inhibitors of cytokine induced GAGs release and simultaneously blocking PGE₂ and NO production triggered by cytokines not only can be effective in reducing the pain and the inflammatory process but also will be able to prevent the progression of the tissue damage and thus will be effective agents for RA pharmacological treatment.

### General description of the invention

This invention relates to a pharmacological treatment of arthritis, in particular osteoarthritis (OA) and rheumatoid arthritis (RA). More specifically, this invention concerns the use of pharmaceutical compositions (drug products) containing effective amounts of compounds of formula (I), as defined below, or their salts or solvate thereof (drug substances), for the treatment of arthritis, OA and RA.

Compounds of formula (I) are known compounds, previously claimed as antivirals or hypoglycaemic agents. We have now surprisingly found for this class of compounds, an unpredictable and outstanding pharmacological activity, previously not reported. Herein, we disclose that compounds of formula (I) are potent inhibitors of IL-1β induced GAG release in articular chondrocytes. As previously discussed, compounds endowed with such an activity will be useful in preventing pathological cartilage degradation. Moreover, we have also disclosed that compounds of formula (I) are endowed with a striking effect in inhibiting simultaneously PGE₂ and NO production, in chondrocytes stimulated with IL-1β. As described in the background, compounds endowed with these pharmacological properties will be effective agents not only in reducing inflammation and pain characterising OA and RA, but also in preventing and reversing the tissue degenerative processes that are responsible for these diseases progression. Accordingly, pharmaceutical compositions (drug substances) containing compounds (drug products) of the present invention will be useful agents for an effective treatment of arthritis, OA and RA.

Within the aims of the present invention the term "treatment" refers to any improvement in clinical symptoms of the diseases, as well as any improvement in the well being of the patients, in particular an improvement manifested by at least one of the following: prevention of the onset of severe disease, decreased joint swelling, decreased pain of the joints, improved mobility, increase in the remission period between acute disease attack, decrease in the time length of the acute attack. In addition, the term treatment in the context of the present invention also refers to slowing of the deterioration of the joints, including the joint tissues and the surrounding tissues, as detected by imaging techniques non-exhaustively including radiography, magnetic resonance imaging (MRI), ultrasound, arthroscopy, etc. (currently known as "structure-modification"), or by a change in the disease biochemical markers.

Within the scope of the present invention the term pharmaceutical composition (drug product) refers to any oral or parenteral dosage form or their pharmaceutical equivalents and/or alternatives (as defined in Note For Guidance on the investigation of bioavailability and Bioequivalence; CPMP/EWP/QWP/1401/98, London, 26 July 2001) particularly suitable for OA and RA treatment, that contains an effective amount of at least one of the active agents, compounds of formula (I), or their salts or solvate thereof and a pharmaceutically acceptable carrier, as defined below, for oral or parenteral administration.

The term mammal includes both humans and all other animal species.

### Detailed description of the invention

The subject matter of the invention is defined by the appended claims.

The active agents of the present invention are the compounds of formula (I), including their corresponding pharmaceutically acceptable salts, solvates prodrugs and bio-precursors as defined below:

### Compounds of formula (I):

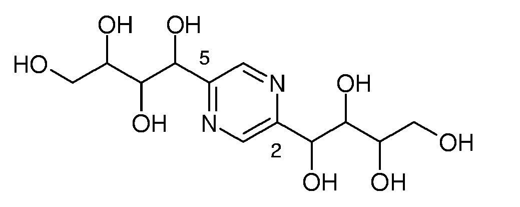

Wherein the 2,5-disubstituted pyrazine ring is intended to be not substituted at positions 3 and 6 as reported in the above formula (I).

The compounds of formula (I) herein described contain several asymmetric centres and thus can give rise to stereoisomers: enatiomers and diastereoisomers, that may be defined in terms of absolute stereochemistry at each stereocentre as ( R ) or ( S ). The present invention includes all such possible isomers as well as their racemic and optically pure forms. Enantiomeric pure forms (enantiomers and diastereoisomers) of compounds of formula (I), encompassed by this invention, are listed in scheme 1:

### Scheme 1

### Compound of formula 1.1

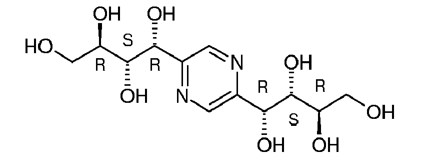

Bis-(1R, 1'R, 2S, 2'S, 3R, 3'R)-1,2,3,4-butanetetrol-1,1'-(2,5-pyrazinediyl); Fructosazine.

### Compound of formula 1.2

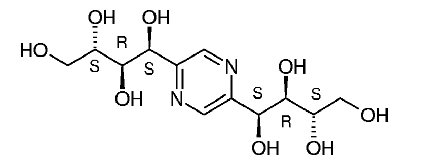

Bis-(1S, 1'S, 2R, 2'R, 3S, 3'S)-1,2,3,4-butanetetrol-1,1'-(2,5-pyrazinediyl).

### Compound of formula 1.3

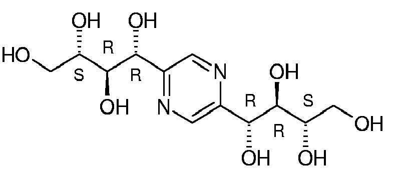

Bis-(1R, 1'R, 2R, 2'R, 3S, 3'S)-1,2,3,4-butanetetrol-1,1'-(2,5-pyrazinediyl).

### Compound of formula 1.4

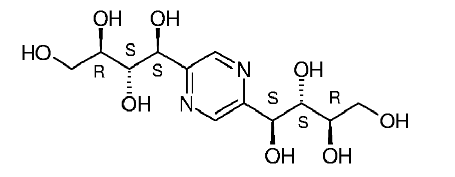

Bis-(1S, 1'S, 2S, 2'S, 3R, 3'R)-1,2,3,4-butanetetrol-1,1'-(2,5-pyrazinediyl).

### Compound of formula 1.5

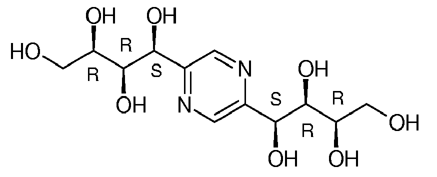

Bis-(1S, 1'S, 2R, 2'R, 3R, 3'R)-1,2,3,4-butanetetrol-1,1'-(2,5-pyrazinediyl).

### Compound of formula 1.6

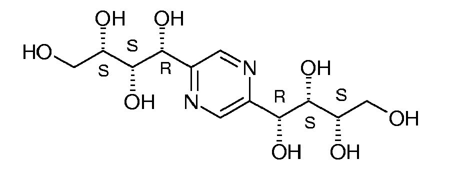

Bis-(1R, 1'R, 2S, 2'S, 3S, 3'S)-1,2,3,4-butanetetrol-1,1'-(2,5-pyrazinediyl); L- Sorbosazine.

### Compound of formula 1.7

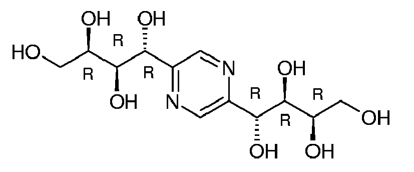

Bis-(1R, 1'R, 2R, 2'R, 3R, 3'R)-1,2,3,4-butanetetrol-1,1'-(2,5-pyrazinediyl); Tagatosazine.

### Compound of formula 1.8

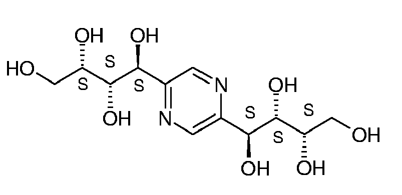

Bis-(1S, 1'S, 2S, 2'S, 3S, 3'S)-1,2,3,4-butanetetrol-1,1'-(2,5-pyrazinediyl).

The compounds of the invention are sufficiently basic to form pharmaceutically acceptable salts, wherein pharmaceutically acceptable salts include but are not limited to: hydrochloride, hydrobromide, sulphate and hydrogensulphate, methansulphonate, maleate, citrate, fumarate and succinate.

Accordingly, the present invention includes within its scope the use of pharmaceutically acceptable salts and solvates of compounds of formula (I) for the treatment of OA and RA.

The present invention includes within its scope pharmaceutical formulations of prodrugs and/or bio-precursors of compound of formula (I) for the treatment of OA and RA.

According to definitions accepted by IUPAC (Glossary of Terms used in Medicinal Chemistry; CG Wermuth et al., Pure and Applied Chemistry, 70, 1129-1143, 1998), a bio-precursor prodrug is a prodrug that does not imply the linkage to a carrier group, but results from a molecular modification of the active principle itself. This modification generates a new compound, able to be transformed metabolically or chemically. The resulting compound being the active principle. A prodrug is any compound that undergoes biotransformation before exhibiting its pharmacological effects. Prodrugs can thus be viewed as drugs containing specialized non-toxic protective groups used in a transient manner to alter or to eliminate undesirable properties in the parent molecule. A double prodrug is a biologically inactive molecule which is transformed in vivo in two steps (enzymatically and or chemically) to the active species.

For example, the compounds of the invention are polyhydroxylated compounds that can form esters with carboxylic acids. Esterases are ubiquitous enzymes that converts, within the organism, the esters into the corresponding alcohol and carboxylic acid. Accordingly, esters of compounds of formula (I) may be suitable pro-drugs of compounds of formula (I), since they can be characterized by a better absorption and distribution profile in comparison with the corresponding parent compound of formula (I), which will be in turn generated anywhere by the esterases.

Esters of compound of formula (I) are represented by compounds of formula (II):

### Compounds of formula (II):

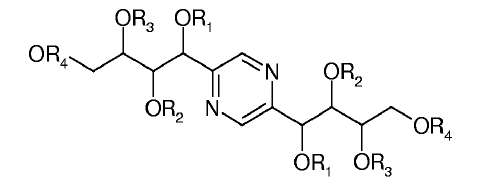

Wherein R₁, R₂, R₃ and R₄ are same or different and independently chosen between a hydrogen atom or an acyl-group (-COR), where R is a C₁-C₅ saturated, linear or branched, alkyl chain or an unsubstituted or substituted phenyl ring or an alkyl-aryl chain; provided that R₁, R₂, R₃ and R₄ are not simultaneously a hydrogen atom. When R is a C₁-C₅ saturated, linear or branched, alkyl chain representative acyl-groups include but are not limited to: acetyl, propanoyl, 2-methylpropanoyl and 3,3-dimethylpropanoyl.

When R is a phenyl ring, suitable substituents in the phenyl ring are halogen atoms (fluoro, chloro), methyl, trifluoromethyl, methoxy and hydroxy. The substituted phenyl ring can be a mono-substituted or di-substituted phenyl ring.

When R is an alkyl-aryl chain it is intended a C₁-C₂ alkyl chain substituted at position 1 or 2 with a substituted or unsubstituted phenyl ring. Suitable substituents for the phenyl ring are halogen atoms (fluoro, chloro), methyl, trifluoromethyl, methoxy and hydroxy. The substituted phenyl ring can be a mono-substituted or di-substituted phenyl ring.

Representative examples of compounds of formula (II) are listed below:
- Acetic acid 2,3,4-triacetoxy-4-[5-(1',2',3',4'-tetracetoxybutyl)-pyrazin-2-yl]-butyl ester, (Formula II where R₁=R₂=R₃= R₄= acetyl) .
- Propionic acid 2,3,4-tripropionyloxy-4-[5-(1',2',3',4'-tetrapropionyloxy-butyl)-pyrazin-2-yl]-butyl ester, (Formula II where R₁=R₂=R₃=R₄= propionyl).
- 3,3-dimethylpropanoic acid 2,3,4-trihydroxy-4-{5-[1',2', 3'-trihydroxy-4'-(3,3-dimethylpropanoyl)butyl)-pyrazin-2-yl}-butyl ester, (Formula II where R₁=R₂=R₃=OH and R₄= 3,3-dimethylpropanoyl).
- Phenyl-acetic acid 2,3,4-trihydroxy-4-[5-(1',2',3'-trihydroxy-4'-phenylacetyl-butyl)-pyrazin-2-yl]-butyl ester, (Formula II where R₁=R₂=R₃=OH and R₄= phenyl-acetyl).
- Acetic acid 2,3-diacetoxy-4-hydroxy-4-[5-(1',2',3'-triacetoxy-4'-hydroxy-butyl)-pyrazin-2-yl]-butyl ester, (Formula II where R₁=R₂=R₃= acetyl and R₄= OH).

### Previous Art

Compounds of the invention are known compounds. Compounds of formula (I) with not specified stereochemistry are described in: Archives of Biochemistry and Biophysics,( M.I. Horowitz; 288, 2, 317-23, 1991) where formation of these compounds was observed in browning reactions of amino sugars; in PCT Int. Appl. WO97/28813 the isolation of mixtures of these compounds from seeds of Eugenia Jambolana Lamarck is described, the use of these compounds as hypoglycemics is claimed; in JP 53 090 401 (1977) these compounds are prepared, and their use for improving tobacco flavour and aroma is claimed.

Compound 1.1 is described in the following documents: Journal of Agricultural and Food Chemistry, 51(21), 6340-6346, 2003, where the formation of compound 1.1 is reported among other products during stressed degradation of amino sugars; in Bioscience, Biotechnology and Biochemistry, 67(2), 295-299, 2003, in Biological and Pharmaceutical Bulletin, 18 (5), 653-8, 1995, and in Chemical & Pharmaceutical Bulletin, 39 (3), 792-4, 1991, compound 1.1 was found to be formed along with other compounds in a study of 2-amino-2-deoxy-D-glucose degradation, aimed at identifying a DNA strand breaking agent; in J. Medicinal Chemistry, 43 (11), 2100-2114, 2000, where compound 1.1 is investigated along with other structurally not related compounds, as template in a molecular modelling study, aimed at identifying a pharmacophore model for HIV-1 integrase; in PCT Int. Appl. WO99/03843 and in PCT Int. Appl. WO99/03842, compound 1.1 is mentioned within a group of compounds acting as hypoglycemics; in Nippon Shokuhin Kogyo Gakkaishi, 37(2), 154-61, 1990, compound 1.1 was isolated along with other compounds in soy sauce; in Carbohydrate research, 184, 67-75, 1988, formation of compound 1.1 in reactions of aldoses and ammonia is described; in JP 45 029 990 and in JP 43 013 469 preparation of compound 1.1 from fructose and its use as antiviral drug is described.

### Compound 1.2, Compound 1.3, Compound 1.4, Compound 1.5, and

Compound 1.8 are described in PCT Int.Appl. WO99/03842, where their use as hypoglycemic agents is claimed.

Compound 1.6 is described: in PCT Int.Appl. WO99/03842, where its use as hypoglycemic agent is claimed; in Carbohydrate Research, 26 (2), 377-84, 1973 where its isolation from a complex mixture of products obtained by ammonolysis of pentacetyl -L-sorbose is described.

Compound 1.7 is described: in PCT Int.Appl. WO99/03842, where its use as hypoglycemic agent is claimed; in JP 45 029 990, where its preparation and use as antiviral is described; in JP 43 013 469 and in J. Organic Chemistry, 31 (7), 2406-7, 1966, where its preparation is described.

Compounds of formula (II) where R is a C₁-C₅ saturated alkyl chain or a phenyl ring are generally claimed as hypoglycemic agents in PCT Int.Appl. WO99/03842. The octaacetyl derivative of compound 1.1, is described in Tetrahedron, 49, 2655-2675, 1993.

### Synthesis of the compounds of the invention

Compounds of formula (I) can be prepared, as pure stereoisomers, starting from the appropriate amino sugar (III), by stirring at a temperature from 0 to 65°C the solution of the amino sugar in an appropriate aqueous or organic solvent, in the presence of a base (Scheme 2).

Suitable solvents for this reaction are alcohols such as: methanol, ethanol or propanol, typically methanol or water. Appropriate bases for this reaction are 24% aqueous ammonia, anhydrous ammonia, or organic bases such as dimethylamine or diethylamine, alkoxide salts such as sodium methoxide or sodium ethoxide. A final chromatographic purification maybe necessary in order to separate the pure compound of formula (I) from reaction by-products.

### Scheme 2

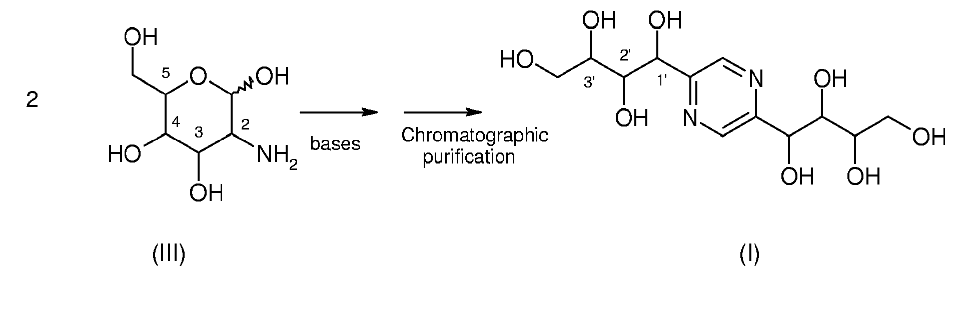

The reaction can be carried out as described in J. Organic Chemistry, 31 (7), 2239-41, 1966 or in Chemical & Pharmaceutical Bulletin, 39 (3), 792-4, 1991. Alternatively, the reaction can be carried out in the presence of lysine as described in Carbohydrate Research, 184, 67-75, (1988).

The stereochemistry at the sugar centres 3, 4, 5 is retained in the obtained compound of formula (I), corresponding respectively at both side chain positions 1', 2' and 3' in compounds of formula (I). Conversely, the stereochemistry at the centre 2 in the amino sugar is lost when compounds of formula (I) are formed. This means that the absolute configuration at the centres 3, 4, 5 of the starting amino sugar will be the absolute configuration respectively at 1', 2', 3' centres of each tetrahydroxybutyl side chain of the obtained compound of formula (1).

For example, both 2-amino-2-deoxy-D-mannose and 2-amino-2-deoxy-D-glucose can be converted into the same compound of formula (I) (compound of formula 1.1, fructosazine), being the two amino sugars different only for the stereochemistry at the C-2 sugar centre while configuration at 3, 4, and 5 is the same (Scheme 3).

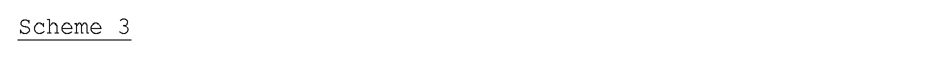

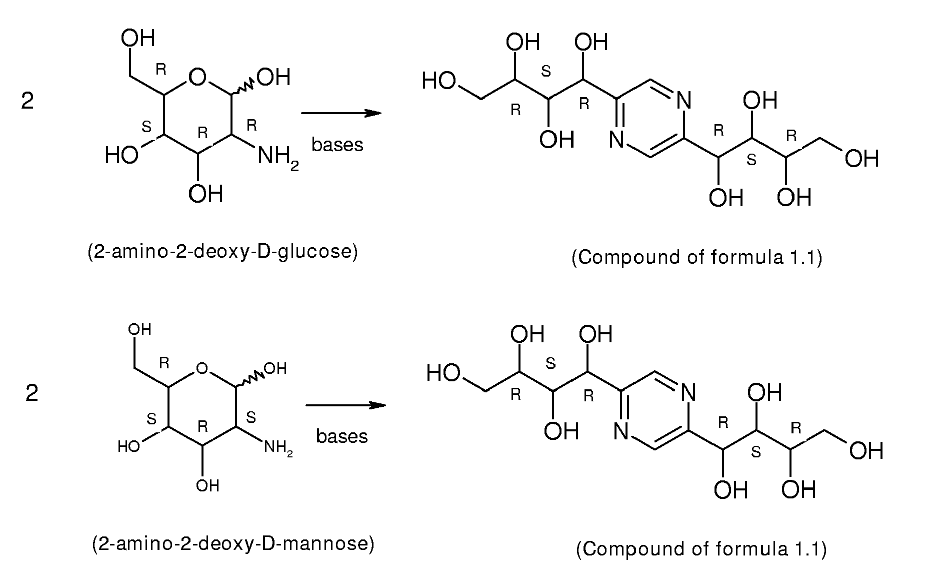

Analogously, reaction of the amino sugar 2-amino-2-deoxy-D-galactose will provide Bis-(1R, 1'R, 2R, 2'R, 3R, 3'R)-1,2,3,4-butanetetrol-1,1'-(2,5-pyrazinediyl), Tagatosazine, compound of formula 1.7 (Scheme 4).

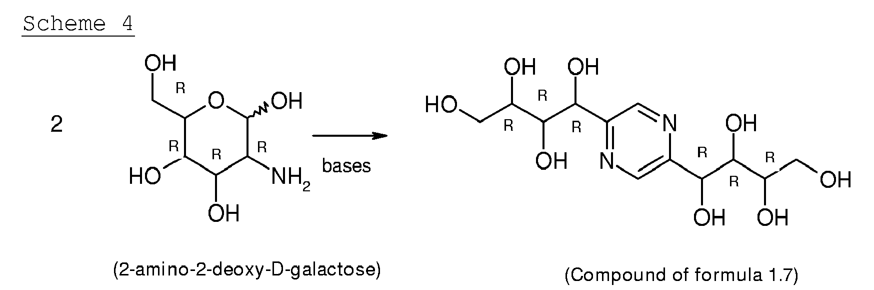

Accordingly, starting from the appropriate amino sugar all the compounds of formula 1.1-1.8 can be prepared. More precisely, compound of formula 1.2 can be obtained from 2-amino-2-deoxy-L-glucose, compound of formula 1.3 can be obtained from 2-amino-2-deoxy-L-altrose, compound of formula 1.4 can be obtained from 2-amino-2-deoxy-D-altrose, compound of formula 1.5 can be obtained from 2-amino-2-deoxy-D-idose or 2-amino-2-deoxy-D-gulose, compound of formula 1.6 can be obtained from 2-amino-2-deoxy-L-idose or 2-amino-2-deoxy-L-gulose.

Starting amino sugars are commercially available compounds or can be prepared from commercial compounds using known methods. Preparations of amino sugars are reported in: Roger W. Jeanloz, Amino Sugars, Academic Press 1969. Several preparations of the mentioned aminosugars are reported in the literature, for example 2-amino-2-deoxy-L-glucose can be prepared as described in Carbohydrate research, 300 (2), 171-174, 1997 or in Bull. Chem. Soc. Japan, 61(4),1291-7, 1988; 2-amino-2-deoxy-D-idose and 2-amino-2-deoxy-D-talose can be prepared as described in J. Org. Chem. 26, 5326, 1961; 2-amino-2-deoxy-L-idose (also 2-amino-2-deoxy L-idropyranose) and 2-amino-2-deoxy-L-gulose are described in Berichte, 90, 2039-49, 1957; 2-amino-2-deoxy-D-allose is described in Berichte, 97(2), 325-30, 1964; preparation of 2-amino-2-deoxy-D-gulose is described in J.Org.Chem., 26, 2153-4, 1961 or in J.Am.Chem.Soc., 79, 2660-1, 1957; preparation of 2-amino-2-deoxy-D-altrose is reported in Nature, 247-8, 1957 and in Experientia, 30 (3), 226-8, 1974.

Compounds of formula (II) can be prepared by reaction of compounds of formula (I) with the appropriate acyl-chloride (R-COCl) or anhydride [(R-CO)₂O], using reaction condition well know in the art (J. March, advanced Organic Chemistry, J.Wiley, 1992). For example, compounds of formula (II) can be obtained by reacting a compound of formula (I) with the acyl-chloride in pyridine or using aqueous NaHCO₃. For the preparation of some compounds of formula (II) selective hydroxyl group protection in compounds of formula (I) may be necessary before the acylation reaction, the subsequent protecting group removal will afford the desired compound of formula (II) or a suitable precursor which can be in turn converted into a compound of formula (II) by a second acylation step. The protective group chemistry is well known to people skilled in the art and proper protecting groups and reaction conditions are reported in T.W. Green, Protective Groups, in Organic Synthesis, J. Wiley, 1991.

The following non-limiting example provides details for the synthesis of compounds of formula (I):

### Example 1: Compound of formula 1.1; Bis-(1R, 1'R, 2S, 2'S, 3R, 3'R)-1,2,3,4-butanetetrol-1,1'-(2,5-pyrazinediyl); Fructosazine

2-Amino-2-deoxy-D-Glucose hydrochloride 100g (0.464 mol) was dissolved in 28% aqueous ammonia (1000 mL), at room temperature. The resulting solution was stirred for 4 weeks at room temperature, ammonia (gas) was slowly bubbled through the mixture during this time. After this time the reaction resulted complete (HPLC); the reaction mixture was then concentrated *in vacuum.* The residue was taken up with methanol and concentrated, then water (250 mL) was added and the pH adjusted at about 7 with 5N HC1. The resulting solution was loaded onto a reverse phase column (250mm x 1m) packed with (Fluka, C18 RP; granulometry: 0.015-0.035 mm). The column is then eluted with demineralised water at a flux of 250 mL/min, the eluate is monitored with an UV detector at 250 nm. The first fractions with UV absorbance were collected and concentrated to provide a residue which was suspended in a small amount of acetonitrile, stirred at r.t. for 15 min., filtered and dried to afford 18 g (20%) of the title compound, fructosazine.

Titled compound: mp.187.8°C; ¹H-NMR (200 MHz; D₂O), δ: 8.75 (2H, s); 5.20 (2H, s); 3.95-3.80 (4H, m); 3.78-3.60 (2H, m). ¹³C-NMR ( D₂O), δ: 67.0 (C-4'), 75.0 (C-3'), 75.5 (C-2'), 77.5 (C-1'), 146.0 (C-3 and C6), 159.1 (C-2 and C-5).

The following compounds are analogously prepared:
- Bis-(1S, 1'S, 2R, 2'R, 3S, 3'S)-1,2,3,4-butanetetrol-1,1'-(2,5-pyrazinediyl; compound of formula 1.2.
- Bis-(1R, 1'R, 2R, 2'R, 3S, 3'S)-1,2,3,4-butanetetrol-1,1'-(2,5-pyrazinediyl); compound of formula 1.3.
- Bis-(1S, 1'S, 2S, 2'S, 3R, 3'R)-1,2,3,4-butanetetrol-1,1'-(2,5-pyrazinediyl); compound of formula 1.4.
- Bis-(1S, 1'S, 2R, 2'R, 3R, 3'R)-1,2,3,4-butanetetrol-1,1'-(2,5-pyrazinediyl); compound of formula 1.5.
- Bis-(1R, 1'R, 2S, 2'S, 3S, 3'S)-1,2,3,4-butanetetrol-1,1'-(2,5-pyrazinediyl); L- Sorbosazine; compound of formula 1.6.
- Bis-(1R, 1'R, 2R, 2'R, 3R, 3'R)-1,2,3,4-butanetetrol-1,1'-(2,5-pyrazinediyl); Tagatosazine; compound of formula 1.7.
- Bis-(1S, 1'S, 2S, 2'S, 3S, 3'S)-1,2,3,4-butanetetrol-1,1'-(2,5-pyrazinediyl); compound of formula 1.8.

### Pharmacological Activity

The compounds of the present invention are potent inhibitors of IL-1β induced GAG release in human articular chondrocytes. As explained in the background, compounds endowed with such an activity are useful in preventing pathological cartilage degradation by inhibiting aggrecan degradation resulting in GAG release. Accordingly, pharmaceutical compositions, pharmaceutical equivalents and/or alternatives of these compounds can prevent the cartilage damage progression and thus can be useful pharmacological agents for OA and RA treatments.

Moreover, the effectiveness of pharmacological agents that can block simultaneously PGE₂ and NO pathways, in preventing typical OA and RA tissues damage along with reducing inflammation and pain has been discussed in depth in the background.

Compounds of the present invention are endowed with a remarkable effect in inhibiting simultaneously PGE₂ and NO production, in chondrocytes stimulated with IL-1β.

Accordingly, the compounds of formula (I), their salts or solvates as well as their prodrugs or bioequivalents, can effectively prevent the cartilage damage progression, the inflammatory processes and the pain that characterise OA and RA, and thus can be useful pharmacological agents for OA and RA treatment.

Quantitative effects in inhibiting IL-1β induced GAG release, PGE₂ and NO synthesis in chondrocytes are reported in Table 1 for the representative compound of formula 1.1. These effects are compared with other compounds recently described in the literature as possible disease modifying drugs for arthritis treatment. SB-242235 (Drug Data report, 19, 11, 1026, 1996) is a p38 MAP Kinase inhibitor which is at present one of the few reported compounds able to block both IL-1β induced PGE₂ and NO synthesis; EGCG is at present one of the few reported compounds able to block IL-1β induced GAG release. However neither SB-242235 nor EGCG are reported to simultaneously inhibit PGE₂ and NO production as well as GAG release.

The compound of the present invention have shown to possess simultaneously all the aforementioned inhibitory activities toward PGE₂, NO and GAG as shown in Table 1

**Table 1**

| Compound | GAG Release inhibitory activity IC₅₀ µM | PGE₂ Synthesis inhibitory activity IC₅₀ µM | NO Synthesis inhibitory activity IC₅₀ µM |
|---|---|---|---|
| Compound 1.1 | 46⁽¹⁾ | 4⁽¹⁾ | 8⁽¹⁾ |
| SB-242235⁽²⁾ | NE⁽³⁾ | 1-3⁽³⁾ | 1.0⁽⁴⁾ |
| EGCG ⁽⁵⁾ | about 100⁽⁶⁾ | >>200⁽⁷⁾ | about 200⁽⁷⁾ |

| | | | |
|---|---|---|---|
| NE: Not effective 1.GAG release, PGE₂ and NO inhibitory activities are assessed after stimulation with 0.3 ng/mL of IL-1β. 2.Osteoarthritis and Cartilage, 2000, 8(6), 434. 3.Human chondrocytes, IL-1β induced inhibition; Osteoarthritis and Cartilage, 2000, 8(6), 434. 4.Bovine articular cartilage, IL-1β induced inhibition; Osteoarthritis and Cartilage, 2000, 8(6), 434. 5..EGCG: epigallocatechin gallate; Arthritis Treatment, WO00/74662. 6.Human articular cartilage, IL-1β induced inhibition; JPET, 308, 767-773, 2004. 7.Human articular chondrocytes, IL-1β induced inhibition, Free Rad.Biol.Med. 33, 1097-1105, 2002 | | | |

Inhibitory effects of compounds of this invention were assessed according to the following *in vitro* assay:

### Primary cell culture

Normal articular chondrocytes were obtained from young New Zealand white rabbits. Shoulders, femoral heads and knees were detached and transferred under a laminar flow hood. The articular cartilage was removed from the articular surfaces under sterile conditions. Tissue fragments were successively digested in an incubator (37°C, 5% CO₂/95% air) with enzymes dissolved in DMEM plus 50 µg/ml gentamicin and sterile filtered. The three-step digestion was carried out with 0.05% testicular hyaluronidase for 15 min, followed by 0.25% trypsin for 30 min, and 0.2% clostridium collagenase for 90 min. At the end of the incubation period, chondrocyte suspension was two-fold diluted with DMEM plus 10% FCS, passed through a 200 µm and a 50 µm nylon sieves to remove debris and undigested matrix particles. Cell suspension was centrifuged at 400 g for 10 min, washed and diluted to 1 x 10⁵ cell/ml, seeded into 24-well culture plates (1 ml/well) in DMEM containing 4.5 g/l glucose supplemented with 10% FCS, 2 mM glutamine and 50 µg/ml gentamicin.

Cells were maintained at 37°C in a humidified 5% CO₂/95% air incubator for a period of 3-4 days by allowing them to adhere to culture plates. Once cell growing started, the culture medium was changed every two days, and the cells reached confluence in 15 days.

### Culture stimulation with interleukin-1ß (IL-1ß)

Confluent cells were washed twice with PBS and stimulated with IL-1β, 0.3 ng/ml, for 24 h. Compounds of the invention were added 20 min before stimulation. The stimulation was performed in DMEM phenol-free, 1 g/l glucose, 50 µg/ml gentamicin, and substituted with 0.1% bovine serum albumin (BSA). At the end of the stimulation period supernatant was stored at -80°C until use.

### Proteoglycan degradation in cell culture

Proteoglycans associated to cell monolayer were quantified with Dimethylmethylene blue (DMB) *assay.* At the end of the stimulation period with IL-1β, culture media were removed and assayed for sulphated glycosaminoglycan (GAG) content using the methachromatic dye 1.9-dimethylmethylene blue (Farndale RW, Sayers CA, Barrett AJ. A direct spectrophotometric microassay for sulphated glycosaminoglycans in cartilage cultures. Conn Tiss Res 1982; 9:247-248). DMB binds to sulphated GAG (S-GAG), which causes a change from blue to purple colour. An aliquot of a sample up to 100 µl was put on a 96-well microtiter plate, mixed with 100 µl of DMB solution. The absorbance was read within 20 min at 590 nm with a HTS7000plus plate reader. The A₅₉₀ value was compared with a standard curve prepared using the same volume of solution containing 1.5 to 15 µg/ml chondroitin 4-sulfate from bovine trachea dissolved in PBS-1%.HCl. The optical density was measured at 590 nm using a plate reader HTS7000plus (Perkin Elmer).

### Nitrite determination

NO production by chondrocytes was measured as nitrite, a stable end product and a reliable indicator of NO synthesis. Nitrites released in the culture supernatant were quantified with the fluorometric method according to Misko (Misko TP, Schilling RJ, Salvemini D, Moore WM, Currie MG. Anal Biochem; 214:11-16, 1993). This assay is based on the reaction of 2,3-diaminonaphthalene (DAN) with nitrite under acidic conditions. The intensity of fluorescence was recorded in a 96-well plate (optiplate, Packard) with a plate reader (HTS7000 plus, Perkin Elmer) with excitation at 360 nm and emission read at 465 nm. Nitrite concentrations were calculated from a standard curve of sodium nitrite ranging from 10 to 100 ng/ml.

### Prostaglandin E₂ (PGE₂) determination

PGE₂ released into the culture medium was determined with a RadioImmunoAssay (RIA) performed according to the instructions of the PGE₂ antiserum manufacturer (Sigma). Briefly: rabbit anti-PGE₂ was diluted to obtain an about fifty per cent binding. Tritium labelled PGE₂, 9000 dpm/sample, was used as a marker. The bound PGE₂ was separated from free PGE₂ by a dextran-coated charcoal suspension sedimented at 2000 g for 15 min. The supernatant was counted in 7 ml of Ultima-Gold MV® and the amount of radioactivity measured in a Packard Tricarb β-counter. Unknown samples were extrapolated from a standard curve using a log-logit curve fit. The standard curve range was 15-500 pg/100 µl.

### Method of Treatment

Compounds of formula (I) or their pharmaceutical equivalents and/or alternatives such as salts, solvates, prodrugs or bioequivalents, can be used in the manufacture of a suitable medication for the therapeutic treatment of arthritis, OA and RA using oral and/or parenteral dosage forms. The method of treating arthritis, RA and OA comprises administering an effective amount of a compound of formula (I) or a pharmaceutical equivalent and/or alternative such as a pharmaceutically acceptable salt, bio-precursor or pro-drug. For the purposes herein, the compounds of formula (I), their pharmaceutical equivalent and/or alternative all have the same dosages and formulations as that of formula (I) and thus are used interchangeably. For all methods of use disclosed herein for the compounds of formula (I) the daily oral dosage regimen will preferably be from about 0.01 to about 100 mg/Kg of total body weight. The daily parenteral dosage regimen will preferably be from about 0.01 to about 10 mg/kg of total body weight. It will also be recognised by one of skill in the art that the optimal quantity and spacing of individual dosages of a compound of formula (I) will be determined by the nature and extent of the condition being treated. In order to use a compound of formula (I), or its bio-precursor, compound of formula (II), or its salt thereof in therapy, it will normally be formulated into a dosage form in accordance with current guidelines and relevant good laboratory and manufacturing practices. This invention, therefore, also relates to a composition suitable for OA and RA treatment, containing an amount of a compound of formula (I), or its pharmaceutical equivalents and/or alternatives and its pharmaceutically acceptable carrier or diluent.

The preferred route of administration for the compounds of the invention is oral, therefore the compound should have suitable permeability across the human intestinal epithelia to allow absorption into the circulation. However, also when the parenteral route is chosen for administration, an effective compound must be sufficiently permeable across biological membranes to reach the site/s of action. The permeability of compounds of formula (I) has been assessed in vitro using the apparent permeability coefficients approach (Papp) (Pharmacokinetics Optimization in Drug Research: biological, physicochemical and computational strategies; Bernard Testa, Han van de Waterbeemd, 2001, Wiley). The TC-7 clone was used instead of the Caco-2 cell line for this assessment, since it was reported that the two human intestinal epithelial cell lines are equivalent as far as drug transport characteristics are concerned and thus can be used interchangeably to predict absorption in humans after oral administration (MC Gres et al., Pharm. Res., 15, 5, 726-33, 1998); the experimental work was carried out as described in the mentioned literature.

An additional requirement for a drug intended for chronic use, is an acceptable dosing regimens (preferably once a day). Such a dosing regimen is attainable if the compound has a suitable clearance and therefore elimination half life from circulation. Thus, compounds that have a greater metabolic stability will ensure a correspondingly longer elimination half life and will be suitable for a once daily administration regimen.

Metabolic stability for the compounds of the invention has been assessed using rat, dog and human liver S9 fractions, using the same methods as described in the literature (R Singh et al., Rapid Commun. Mass Spectrom., 10, 9, 1019-26, 1996).

As representative not limiting example, data for compound 1.1 are reported in table 2.

Permeability and metabolic stability data are the average of two experiments.

**Table 2**

| Example | A-B permeability (pH: 6.5/7.4) 10⁻⁶ cm/sec | B-A permeability (pH: 6.5/7.4) 10⁻⁶ cm/sec | *In vitro* metabolism Parent remaining (%) Test concentration 1µM | | |
|---|---|---|---|---|---|
| | | | Human | Dog | Rat |
| Compound 1.1 | 112.9 | 72.9 | 108 | 96 | 110 |

The above data indicate that compounds of the invention are permeable across the human intestinal epithelia and will be absorbed after oral administration. In addition, the compounds permeability will allow their distribution outside of blood compartment to reach the site of action. Finally, their metabolic stability is as such that they will be suitable candidates for once a day oral administration regimen.

The compounds of the invention can be formulated for OA and RA treatment, in a wide variety of oral dosage forms, such as capsules, tablets, pills, dispersible granules. Suitable carriers include but are not limited to magnesium carbonate, magnesium stearate, talc, lactose, methylcellulose, sodium carboxymethyl cellulose.

Techniques used to prepare oral formulations are the conventional mixing, granulation and compression or capsules filling. Other forms suitable for oral administration include emulsions, syrups and aqueous solutions. Emulsions can be prepared using emulsifying agents for example lecithin, propylene glycol or sorbitan monooleate. Aqueous solutions can be prepared by dissolving the active component in water and adding suitable colorants, flavours, stabilising agents.

Compounds of the invention have displayed a suitable solubility in water at pH 7.4, for example compound 1.1 can be dissolved in water up to 0.1 M concentration.

This good water solubility not only highlights how compounds of the invention can be suitable for oral administration of compositions as aqueous solution, but especially indicates how compounds of formula (I) can be appropriately used for the preparation of parenteral dosage forms.

The compounds of the present invention maybe formulated for parenteral administration (e.g., by injection or by continuous infusion) and maybe presented in unit dose forms, for example in ampoules or pre-filled syringes. The drug product may be prepared using aqueous vehicles solutions and/or oily emulsions (particularly suitable for compounds of formula II).

## Claims

1. The use of a compound of formula (I): 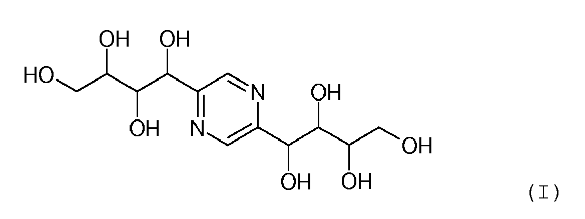 or of any of its single diastereoisomers or enantiomers, racemic or non-racemic mixture of isomers or of pharmaceutical alternative and/or equivalent compounds thereof, including their prodrugs, and/or bio-precursors, and/or salts for preparing a medicament for treating osteoarthritis in mammals.

2. The use of a compound of claim 1 for preparing a medicament for treating Rheumatoid Arthritis in mammals.

3. The use of a compound of formula (II) 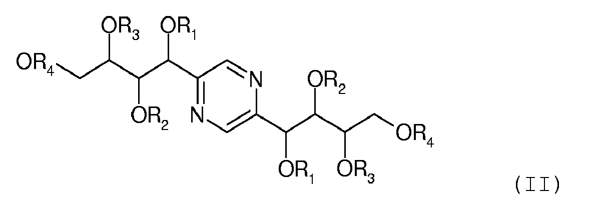 wherein R₁, R₂, R₃ and R₄ are the same or different and are independently selected from the group consisting of a hydrogen atom and an acyl-group (-COR), where in -COR R is a C₁-C₅ saturated, linear or branched, alkyl chain or an unsubstituted or substituted phenyl ring or an alkyl-aryl chain, provided that R₁, R₂, R₃ and R₄ are not simultaneously a hydrogen atom, or of any individual diastereoisomer or enantiomer, racemic or non-racemic mixture of isomers or pharmaceutically acceptable salts thereof, or of pharmaceutically alternative and/or equivalent compounds thereof, including their prodrugs and/or bio-precursors and/or salts, for preparing a medicament for treating osteoarthritis in mammals.

4. The use of claim 3, wherein in formula (II) R₄ is -COR and R₁, R₂ and R₃ are all H.

5. The use according to claims 3 or 4, wherein said acyl group (-COR) is selected from the group consisting of acetyl, propanoyl, 2-methylpropanoyl and 3,3-dimethylpropanoyl.

6. The use according to claims 3 or 4, wherein in at least one of the acyl groups (-COR) R is a phenyl ring unsubstituted or mono- or di-substituted with halogen atoms, methyl, trifluoromethyl, methoxy or hydroxy.

7. The use according to claims 3 or 4, wherein in at least one of the acyl groups (-COR) R is an alkyl-aryl chain consisting of a C₁-C₂ alkyl chain substituted at positions 1 or 2 with a substituted or unsubstituted phenyl ring.

8. The use according to claim 7, wherein said phenyl ring in the alkyl-aryl chain is mono- or di-substituted with halogen atoms, methyl, trifluoromethyl, methoxy or hydroxy.

9. The use of a compound as defined in any of claims 3 to 8 for preparing a medicament for treating Rheumatoid Arthritis in a mammal.

10. The use according to any of claims 1 to 9, wherein said medicament further includes at least one pharmaceutically acceptable carrier.

11. The use according to any of claims 1 to 10, wherein said medicament is in a dosage form for oral and/or parenteral administration.

12. The use of a compound as defined in any of claims 1 and 3 to 8 for preparing a medicament for inhibiting IL-1β induced PGE₂ synthesis in a mammal.

13. The use of a compound as defined in any of claims 1 and 3 to 8 for preparing a medicament for inhibiting IL-1β induced NO synthesis in a mammal.

14. The use according to claims 12 or 13, wherein said medicament is for the treatment of a disease selected from systemic Lupus erythematosus, progressive systemic sclerosis, ulcerative colitis, Crohn's disease, inflammatory bowel syndrome, septic shock and neurodegeneration.

15. The use of a compound as defined in any of claims 1 and 3 to 8 for preparing a medicament for treating inflammation or pain in a mammal.
